(19) Europäisches Patentamt / European Patent Office / Office européen des brevets

(11) **EP 3 940 597 A1**

(12) **EUROPEAN PATENT APPLICATION**

(43) Date of publication:
**19.01.2022 Bulletin 2022/03**

(51) International Patent Classification (IPC):
***G06N 3/00*** *(2006.01)*

(21) Application number: **20186331.3**

(52) Cooperative Patent Classification (CPC):
**G06N 3/00;** G16H 50/20

(22) Date of filing: **16.07.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Koninklijke Philips N.V.**
**5656 AG Eindhoven (NL)**

(72) Inventors:
• **PATIL, Ravindra**
**5656 AE Eindhoven (NL)**
• **MYSORE SIDDU, Dinesh**
**5656 AE Eindhoven (NL)**
• **SUSAIYAH, Allmin Pradhap Singh**
**5656 AE Eindhoven (NL)**
• **ANAND, Shreya**
**5656 AE Eindhoven (NL)**
• **PANDYA, Maulik**
**5656 AE Eindhoven (NL)**
• **BUSSA, Nagaraju**
**5656 AE Eindhoven (NL)**

(74) Representative: **Philips Intellectual Property & Standards**
**High Tech Campus 52**
**5656 AG Eindhoven (NL)**

(54) **SELECTING A TRAINING DATASET WITH WHICH TO TRAIN A MODEL**

(57) According to an aspect, there is provided a computer implemented method in a central server of selecting a training dataset with which to train a model using a distributed machine learning process, wherein the training dataset is to comprise medical data that satisfies one or more clinical requirements and wherein training data in the training dataset is located at a plurality of clinical sites. The method comprises requesting (302) from each of the clinical sites, metadata describing features of matching data at the respective clinical site that satisfies the one or more clinical requirements. The method then comprises determining (304), from the metadata, a measure of variation of the features of the matching data. Based on the measure of variation, the method then comprises selecting (306) training data for the training dataset from the matching data using the metadata.

Fig. 4

**EP 3 940 597 A1**

**Description**

FIELD OF THE INVENTION

Embodiments herein relate to training a model using a distributed machine learning process.

BACKGROUND OF THE INVENTION

Learning from large volumes of patient data can greatly increase capacity to generate and test hypotheses about healthcare. To capture and use the knowledge contained in large volumes of patient data, predictive models are used. Models can be trained using machine learning processes on large volumes of data from patients who have been treated previously. Models trained in this manner have the potential to be used to make predictions in many areas of medicine, such as image segmentation and diagnosis, amongst others. Such models may be used to better personalise healthcare.

One of the hurdles in enabling personalised medicine through the use of models trained using machine learning processes, is obtaining sufficient patient data to train the models. The data from one single hospital is unlikely to be sufficient to develop models which can be used on a wide variety of patients (e.g. which may be spread across the globe). However, to get the data from different hospitals and patient groups can take a long time which increases the time from planning to deployment of the models. In the Deep Learning domain, the performance of models improves with increasing numbers of training data samples. Thus, to ensure the best possible models to aid physicians, the performance of the models can be actively improved with more data. Combining data originating from multiple clinical sites (e.g. hospitals, doctors' surgeries etc) can be difficult however due to ethical, legal, political, and administrative barriers associated with data sharing. One way of mitigating such issues is by training a model using a distributed machine learning process, such as, for example, a Federated Learning process, as described in the paper by Bonawitz et al. 2019 entitled "Towards Federated Learning at Scale: System Design". Distributed learning enables models to be trained using data from different clinical sites without the data leaving the premises.

SUMMARY OF THE INVENTION

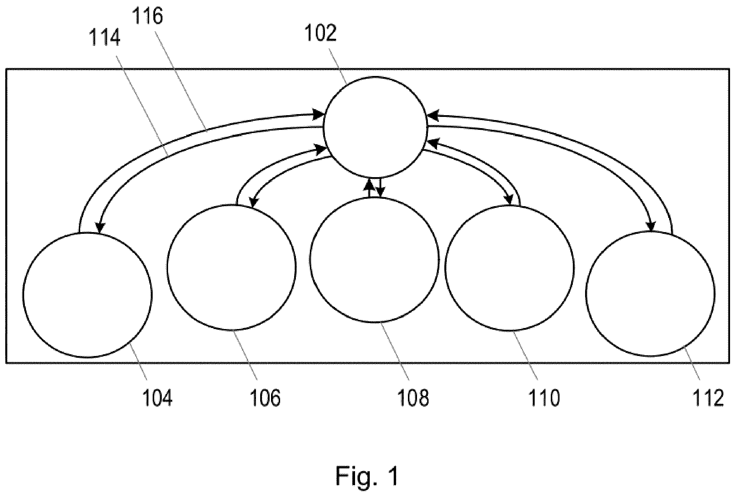

As noted above, distributed machine learning processes can be used to train models (otherwise known as "machine learning models") on training data located at different sites, without the training data needing to be moved from the respective sites. The skilled person will be familiar with distributed learning and distributed learning processes such as federated machine learning, however, this is illustrated briefly in Fig. 1 which shows a central server 102 in communication with a plurality of clinical sites (e.g. healthcare data centres HDCs), 104 to 112. The central server co-ordinates training of a model using a distributed learning process using training data located at each of the clinical sites 104 to 112. The central server holds a "global" or central copy of the model and may send 114 information about the global model, e.g. such as parameters enabling a local copy of the model to be created, to each clinical site. Each clinical site may then create a local copy of the model and train its local copy on training data at the respective clinical site. Each clinical site 104 to 112 may then send 116 an update to one or more parameters of the model to the central server. The central server combines the updates, for example, through averaging, from the respective clinical sites to update the global model. This allows a global model at a central server 102 to be trained e.g. updated and improved, based on training data at a plurality of clinical sites 104 to 112, without the data having to leave the respective clinical site.

It is an object of embodiments herein to improve on processes for training models on medical data using distributed machine learning.

Thus, according to a first aspect, there is provided a computer implemented method in a central server of selecting a training dataset with which to train a model using a distributed machine learning process, wherein the training dataset is to comprise medical data that satisfies one or more clinical requirements and wherein training data in the training dataset is located at a plurality of clinical sites, the method comprising: requesting from each of the clinical sites, metadata describing features of matching data at the respective clinical site that satisfies the one or more clinical requirements; determining, from the metadata, a measure of variation of the features of the matching data; and based on the measure of variation, selecting training data for the training dataset from the matching data using the metadata.

In this manner, the central server may select a varied training dataset from training data located at a different sites, without the training data having to be transmitted to the central server itself. Thus instead of the central server being effectively "blind" to the data on which the model is trained (because the training data is located at and selected by the clinical sites), the central server is able to select and coordinate training data. Thus training may be performed in a more systematic way. By selecting the data based on variation in the data samples, a more varied and representative training dataset may be selected. The resulting model may thus be more generalised. In summary, in this way, a more robust global model may be built by pre-analysing the local data. Coupled with the distributed nature of the training process, embodiments herein provide the above advantages whilst maintaining patient privacy.

According to a second aspect there is an apparatus for selecting a training dataset with which to train a model using a distributed machine learning process, wherein the training dataset is to comprise medical data that satisfies one or more clinical requirements and wherein training data in the training dataset is located at a plurality of clinical sites. The apparatus comprises a memory comprising instruction data representing a set of instructions, and a processor configured to communicate with the memory and to execute the set of instructions. The set of instructions, when executed by the processor, cause the processor to request from each of the clinical sites, metadata describing features of matching data at the respective clinical site that satisfies the one or more clinical requirements; determine, from the metadata, a measure of variation of the features of the matching data; and based on the measure of variation, select training data for the training dataset from the matching data using the metadata.

According to a third aspect there is a computer program product comprising a computer readable medium, the computer readable medium having computer readable code embodied therein, the computer readable code being configured such that, on execution by a suitable computer or processor, the computer or processor is caused to perform the method of the first aspect.

These and other aspects will be apparent from and elucidated with reference to the embodiment(s) described hereinafter.

BRIEF DESCRIPTION OF THE DRAWINGS

Example embodiments will now be described, by way of example only, with reference to the following drawings, in which:

Fig. 1 illustrates a distributed learning process for training a machine learning model;
Fig. 2 illustrates an apparatus according to some embodiments herein;
Fig. 3 illustrates a method according to some embodiments herein;
Fig. 4 illustrates a second machine learning model according to some embodiments herein;
Fig. 5 illustrates a process according to some embodiments herein; and
Fig. 6 illustrates a method of training a model using a machine learning process according to some embodiments herein.

DETAILED DESCRIPTION OF EMBODIMENTS

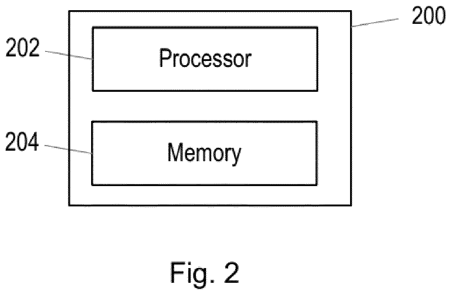

Turning now to Fig. 2 in some embodiments there is an apparatus (e.g. system) 200 for use in selecting a training dataset with which to train a model using a distributed machine learning process, according to some embodiments herein. Generally, the apparatus may form part of a computer apparatus or system e.g. such as a laptop, desktop computer or other computing device. In some embodiments, the apparatus 200 may form part of a distributed computing arrangement or the cloud.

The apparatus comprises a memory 204 comprising instruction data representing a set of instructions and a processor 202 (e.g. processing circuitry or logic) configured to communicate with the memory and to execute the set of instructions. Generally, the set of instructions, when executed by the processor, may cause the processor to perform any of the embodiments of the method 300 as described below.

Embodiments of the apparatus 200 may be for use in selecting a training dataset with which to train a model using a distributed machine learning process, wherein the training dataset is to comprise medical data that satisfies one or more clinical requirements and wherein training data in the training dataset is located at a plurality of clinical sites. More specifically, the set of instructions, when executed by the processor, cause the processor to: request from each of the clinical sites, metadata describing features of matching data at the respective clinical site that satisfies the one or more clinical requirements; determine, from the metadata, a measure of variation of the features of the matching data; and based on the measure of variation, select training data for the training dataset from the matching data using the metadata.

The processor 202 can comprise one or more processors, processing units, multi-core processors or modules that are configured or programmed to control the apparatus 200 in the manner described herein. In particular implementations, the processor 202 can comprise a plurality of software and/or hardware modules that are each configured to perform, or are for performing, individual or multiple steps of the method described herein. The processor 202 can comprise one or more processors, processing units, multi-core processors and/or modules that are configured or programmed to control the apparatus 200 in the manner described herein. In some implementations, for example, the processor 202 may comprise a plurality of (for example, interoperated) processors, processing units, multi-core processors and/or modules configured for distributed processing. It will be appreciated by a person skilled in the art that such processors, processing units, multi-core processors and/or modules may be located in different locations and may perform different steps and/or different parts of a single step of the method described herein.

The memory 204 is configured to store program code that can be executed by the processor 202 to perform the method described herein. Alternatively or in addition, one or more memories 204 may be external to (i.e. separate to or remote from) the apparatus 200. For example, one or more memories 204 may be part of another device. Memory 204 can be used to store the metadata, the measure of variation and/or any other information or data received, calculated or determined by the processor 202 of the apparatus 200 or from any interfaces, memories or devices that are external to the apparatus 200. The processor 202 may be configured to control the memory 204 to store the metadata, the measure of variation and/or the any other information or data received, calculated or determined by the processor 202.

In some embodiments, the memory 204 may comprise a plurality of sub-memories, each sub-memory being capable of storing a piece of instruction data. For example, at least one sub-memory may store instruction data representing at least one instruction of the set of instructions, while at least one other sub-memory may store instruction data representing at least one other instruction of the set of instructions.

It will be appreciated that Fig. 2 only shows the components required to illustrate this aspect of the disclosure and, in a practical implementation, the apparatus 200 may comprise additional components to those shown. For example, the apparatus 200 may further comprise a display. A display may comprise, for example, a computer screen, and/or a screen on a mobile phone or tablet. The apparatus may further comprise a user input device, such as a keyboard, mouse or other input device that enables a user to interact with the apparatus, for example, to provide initial input parameters to be used in the method described herein. The apparatus 200 may comprise a battery or other power supply for powering the apparatus 200 or means for connecting the apparatus 200 to a mains power supply.

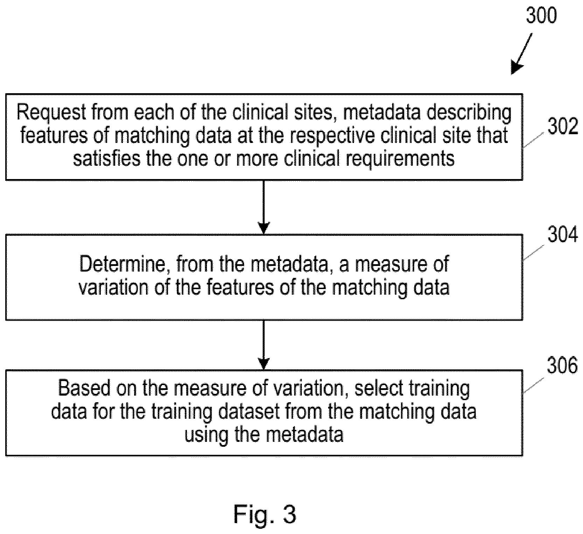

Turning to Fig. 3, there is a computer implemented method in a (e.g. performed by) a central server 300 for use in selecting a training dataset with which to train a model using a distributed machine learning process, wherein the training dataset is to comprise medical data that satisfies one or more clinical requirements and wherein training data in the training dataset is located at a plurality of clinical sites. Embodiments of the method 300 may be performed, for example, by an apparatus such as the apparatus 200 described above.

Briefly, in a first step, the method 300 comprises: requesting 302 from each of the clinical sites, metadata describing features of matching data at the respective clinical site that satisfies the one or more clinical requirements. In a second step the method comprises determining 304, from the metadata, a measure of variation of the features of the matching data. In a third step the method comprises, based on the measure of variation, selecting 306 training data for the training dataset from the matching data using the metadata.

In this way metadata may be used to enable the central server to select, from the suitable matching data, an appropriate training dataset with which to train the model, based on the variation in the selected training dataset as determined from the metadata. This allows the central server more control over the training and completeness of the training data, without compromising patient confidentiality (e.g. by sending the data to the central server).

In more detail, as noted above methods and systems herein relate to selecting a training dataset with which to train a model such as any of the models described below, using a distributed learning process. Distributed learning processes were described above with respect to Fig. 1 and the detail therein will be understood to apply to embodiments of the apparatus 200 and the method 300. Examples of distributed learning processes include, but are not limited to Federated Learning and Distributed Data Parallelism methods.

In some embodiments the apparatus 200 may comprise a server or computing apparatus that co-ordinates the training performed by the servers at the plurality of clinical sites, in other words, a "central server" (note that "central" in this context doesn't necessarily infer a geographical position relative to the clinical sites). Herein the method 300 may be performed or initiated by a user, company or any other designer or orchestrator of the training process, e.g. using the apparatus 200. Using terminology commonly associated with distributed learning processes, the central server (e.g. such as an apparatus 200) may comprise the "master" of the distributed learning process and the plurality of clinical sites may comprise "workers" or nodes.

In this context a clinical site may comprise a server (e.g. a "clinical server") or a datacentre associated with a hospital, a surgery, a clinic, or any other medical facility. A clinical site may comprise, for example, a datacentre such as a Hospital Data Centre (HDC) or any other computing site suitable for storing medical data.

As noted above, the training dataset is for training a model using a distributed machine learning process. The training dataset may be for use in training the model to perform a task, e.g. a task related to medical data. For example, the training may be performed to train the model to classify medical data, segment medical data (e.g. medical images) or to perform any other type of task that may be performed on medical data by a model.

In more detail, the model may comprise any type of model that can be trained using a machine learning process e.g. a machine learning model. Examples of models include, but are not limited to neural networks, deep neural networks such as F-Nets, U-Nets and Convolutional Neural Networks, Random Forest models and Support Vector Machine (SVM) models.

The skilled person will be familiar with machine learning and machine learning models, but briefly, machine learning can be used to find a predictive function for a given dataset; the dataset is typically a mapping between a given input to an output. The predictive function (or mapping function) is generated in a training phase, which involves providing example inputs and ground truth (e.g. correct) outputs to the model. A test phase comprises predicting the output for a given input. Applications of machine learning include, for example, curve fitting, facial recognition and spam filtering.

In some embodiments herein, the model comprises a neural network model, such as a deep neural network model. The skilled person will be familiar with neural networks, but in brief, neural networks are a type of machine learning model that can be trained to predict a desired output for given input data. Neural networks are trained by providing training data comprising example input data and the corresponding "correct" or ground truth outcome that is desired. Neural networks comprise a plurality of layers of neurons, each neuron representing a mathematical operation that is applied to the input data. The output of each layer in the neural network is fed into the next layer to produce an output. For each piece of training data, weights associated with the neurons are adjusted (e.g. using processes such as back propagation and/or gradient decent) until the optimal weightings are found that produce predictions for the training examples that reflect the corresponding ground truths.

In this context medical data may comprise any type of data that can be used, produced and/or obtained in a medical setting, including but not limited to: clinical diagnostic data, such as patient vital signs, or physiological parameters, medical images, medical files (e.g. such as patient records), and/or outputs of medical machines (e.g. operational or diagnostic data from medical equipment).

Generally the training dataset may be for use in training the model to take as input one or more of the types of medical data described above and provide an output. In embodiments herein, the model may be trained to output, for example, a patient diagnosis based on the input medical data. In embodiments where the medical data comprises a medical image, the model may be trained to output, for example, a segmentation of the medical image, a location of a feature of interest in the medical image, or a diagnosis based on the medical image. The skilled person will appreciate however that these are merely examples, and that the model may be trained to take different types of medical data as input and provide different types of outputs to the examples provided above.

The training data is to satisfy one or more clinical requirements. The one or more clinical requirements may relate to the (intended) input parameters of the model. For example if the model is to be trained to perform a first task on a first type of medical image, then the one or more clinical requirements may relate to the first type of image and the characteristics that the first type of image should have in order to be (validly) input into the model. In other words the clinical requirements comprise requirements for the model training or model building. Examples of clinical requirements include but are not limited to parameters such as: Histopathology, Tumor Progression, Tumor Size, etc.

In step 302 the method 300 comprises requesting from each of the clinical sites, metadata describing features of matching data at the respective clinical site that satisfies the one or more clinical requirements. For example the request may comprise a (database) query comprising the clinical requirements e.g. a query requesting details of data that satisfies the clinical requirements.

The method may then comprise receiving the requested metadata (e.g. the results of the request/query) from each of the clinical sites. The metadata received by the central server may comprise, for example, a list of enumerated metadata, each item in the list corresponding to a piece of medical data at the respective clinical site that satisfies the clinical requirements.

The metadata may comprise fields (e.g. features, data types, parameters, entries etc) related to the clinical requirements. The metadata may comprise further fields describing other features of the matching data. In other words, the features in the metadata may comprise different features to those specified in the clinical requirements. For example, parameters unrelated to the clinical requirements (e.g if the clinical requirements comprise a tumor size of a particular type of tumor, the metadata may comprise other features, such as age, gender, and ethnicity of the patient, or other features related to e.g. underlying health conditions of the patient). Use of a wide variety of features in this manner may enable a training dataset to be selected that encompasses a wide variety of different patient characteristics, and thus includes examples of training data that satisfies the clinical requirements derived from a wide range of different patients. This improving the training of the resulting model.

An example of three lines of metadata from a model trained to detect whether a patient has had a stroke is given below. In this example, a clinical requirement for training data is that the slice thickness should be adequate enough such as not to miss any bleeds.

| # | Patient ID | Scan type | Recon.Kernel | KVP | Sequence | Slice Thickness | Contrast | SliceCount | Disease |
|---|---|---|---|---|---|---|---|---|---|
| 1 | P1 | CT | - | Yes | Stroke | 2.5 | Sharp | 120 | 90 |
| 2 | P1 | MRI | Mo | - | Stroke | 1 | - | - | 93 |
| 3 | P10 | CT | - | No | Stroke | 5 | Medium | 110 | 85 |

It will be understood, however that the metadata and the features in the metadata given in the example above are merely an example and that the metadata may comprise many different types of features describing different aspects of the data and/or the patient.

The metadata may be anonymised or de-identified so as to comprise features that cannot be used to identify the patient.

In step 304 the method comprises determining a measure of variation of the features of the matching data. For example, in some embodiments, the measure of variation may comprise a measure of heterogeneity of the features in the metadata, e.g. a measure of diversity of the features (or feature/parameter values). As noted above, the more heterogeneous the features of the matching data are, the more likely that the dataset as a whole will capture the variation seen in global human populations. Models trained on heterogeneous datasets are likely to make better predictions than those trained on less heterogeneous datasets.

The skilled person will appreciate that other measures of variation may also be used, such as for example, measures of homogeneity (in which case, the training data for the training dataset would be selected so as to minimise the homogeneity), or measures of entropy. Some examples of measures of variation include, for example, Dykstra - Parsons Coefficient, and the t-test.

In step 306 the method comprises, based on the measure of variation, selecting training data for the training dataset from the matching data, using the metadata.

In this way, a training dataset is selected, comprising training data located at (e.g. distributed between) a plurality of clinical sites. The training data selected from an individual clinical site to form part of the training dataset may be referred to herein as a "local batch" of data. The full training dataset comprising all the local batches across the plurality of clinical sites may be referred to herein as a "global batch".

In some embodiments, the step of selecting training data for the training dataset from the matching data using the metadata comprises selecting training data from the matching data at the plurality of clinical sites so as to increase the measure of variation of the features in the resulting training dataset (global batch).

In some embodiments, the method may further comprise selecting the training data from the matching data at the plurality of clinical sites so as to obtain an even representation between different data types, e.g. different ground truth labels or classifications in the training dataset. In other words, the training data for the training dataset may be selected so as to obtain a heterogeneous training dataset (e.g. heterogeneous within a tolerance limit), whilst also ensuring even representation of data samples that suffice the need for model development. For example, training may be further improved by having a training dataset with sufficient numbers of training examples in each label (e.g. output) category.

In some embodiments, the measure of variation of the features of the matching data is determined for matching data at each respective clinical site. The step of selecting training data for the training dataset may then comprise selecting the training data from the matching data at the respective clinical site so as to increase the measure of variation of the training data selected from the respective clinical site. Thus the training data selected from each clinical site (e.g. each local batch) may be selected so as to increase the measure of variation (e.g. heterogeneity) of training data selected at each clinical site. In other words, in embodiments where the measure of variation comprises a measure of heterogeneity, a heterogeneity checker may be used to check for local heterogeneity of the local batches.

In some embodiments, the measure of variation of the features of the matching data is determined for matching data across all of the plurality of clinical sites (e.g. for the global batch). The step of selecting the training data from the matching data may then comprise selecting the data across all the plurality of clinical sites so as to increase the measure of variation across the training dataset as a whole. In such a manner, lack of variation in the matching data from one clinical site may be compensated for by matching data from another clinical site. In other words, in embodiments where the measure of variation comprises a measure of heterogeneity, a heterogeneity checker may be used to check for global heterogeneity of the global batch.

In some embodiments, the method may comprise supplementing the training dataset with augmented training data so as to increase the variation of the selected training dataset. Augmented e.g. synthetic training data may be used to increase the variation of the training data at an individual clinical site (e.g. local batch) or to increase the variation of the training dataset as whole (e.g. global batch). The skilled person will be familiar with methods of creating augmented or synthetic medical data. For example, synthetic medical images may be created using techniques such as the synthetic minority oversampling technique (SMOTE), or using Generative adversarial networks (GAN)s, and/or data imputation techniques.

In some embodiments, the measure of heterogeneity is determined using a second machine learning model that takes the features in the metadata as input and outputs the measure of heterogeneity. The second machine learning model may output a list comprising a subset of the matching training data that optimises/maximises the heterogeneity (compared to other possible subsets of the matching training data).

For example, the second machine learning model may take as input, feature values in the metadata, $x_n$, and determine a combination of $x_n$ that optimally flattens a linear function $f(x)=x'\beta+b$, wherein $\beta$ comprises the gradient of the function and b comprises an offset. In this context $x_n$ comprises an entry in the metadata corresponding to a piece of matching training data.

In some embodiments, the second machine learning model is configured to determine $f(x)$ with the minimal norm value $(\beta'\beta)$ according to a convex optimization problem wherein the function:

$$J(\beta)=1/2(\beta'\beta);$$

is to be minimized. In this equation, $\beta'$ is the transpose of $\beta$.

This may be subject to all residuals having a value less than $\varepsilon$. In equation form such a constraint may be written:

$$\forall n: |yn-(xn'\beta+b)|\leq\varepsilon$$

Also, to deal with otherwise infeasible constraints, non-linear kernels may be employed (such as polynomial and Gaussian based on the different type of data in question).

In some embodiments, the second machine learning model may comprise a support vector regression model. The skilled person will appreciate that this is merely an example however and that the second machine learning model may also comprise other types of machine learning model.

In this manner, an optimal combination of training data examples may be selected, from all of the matching data across the clinical sites. In this way it is possible to make sure that heterogeneity is well within a given (e.g. predetermined) tolerance limit and make sure that data is well balanced with normal, positive cases. As such, the second machine learning model may provide the feedback on how the dataset can be improved, thereby providing details on which training data examples to select.

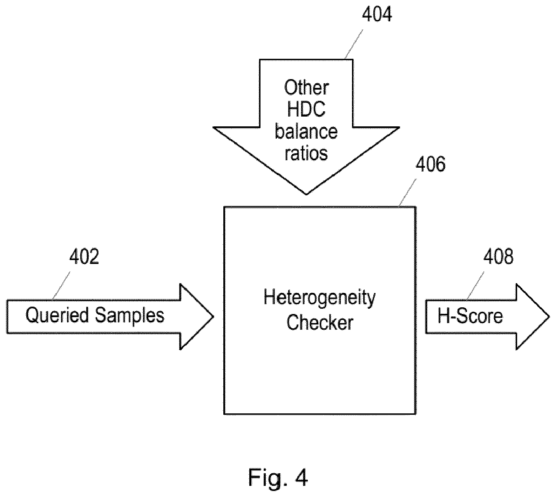

An embodiment of the second machine learning model is illustrated in Fig. 4. In this embodiment, the second machine learning model 406 (otherwise referred to herein as a Heterogeneity checker) comprises a ML based support vector regression model, which considers (e.g. takes as input) feature inputs 402 such as

- Age, Gender
- Disease Pathology
- Different associated conditions/ co morbidity
- Image scan types and the settings (Slice thickness, KVMA, Kernel type)
- DICOM attributes such as reconstruction kernel, vendor type etc.

In this embodiment, the second machine learning model may further take as input other Healthcare Data Centre (HDC) balance ratios 404. A HDC balance ratio may comprise a parameter that aids understanding of data ratios across different HDC's. This may help in the heterogeneity computation, as well as feedback mechanism on where it has to be corrected (Eg Diseased vs Non Diseased subject pool being 0.6 or 0.8, for example).

Formulation: In this embodiment, $x_n$ is a multivariate set (e.g. the features described above) of $N$ observations with observed response values $y_n$, (e.g. ground truth values) with heterogeneity score (H Score). The heterogeneity score may vary depending on how the system is designed. For example, it may be designed such that H comprises a normalized value between 0 and 1.

The objective of the second machine learning model is to find the linear function $f(x)=x'\beta+b$, and ensure that it is as flat as possible, by finding $f(x)$ with the minimal norm value $(\beta'\beta)$. As described above, this may be formulated as a convex optimization problem to minimize

$$J(\beta)=1/2(\beta'\beta)$$

subject to all residuals having a value less than $\varepsilon$, according to: $\forall n:|yn-(xn'\beta+b)|\leq\varepsilon$ As noted above, in this embodiment, to deal with otherwise infeasible constraints, non linear kernels may be employed, dependent on the data, (such as polynomial and Gaussian). The second machine model outputs a heterogeneity score 408.

In this way a training set may be selected with optimal variation of training data, from the available data that matches the clinical requirements.

Turning back to the method 300, once the training data has been selected, the method 300 may further comprise training the model using the selected training dataset. The model may be trained according to a distributed learning process as described above. The central server may instruct each clinical site to create a local copy of the model and train the local copy of the model on the training data in the training dataset selected from the respective clinical site. For example, the central server may send an indication of which of the matching data at the respective clinical site should be used by that clinical site to train the model.

The method may then proceed according to the distributed learning process. For example, the method 300 may further comprise combining the results of the training according to the distributed learning process. The skilled person will be familiar with combining results of training at different sites according to a distributed learning process, but as an example, each clinical site may send updates to parameters in the model resulting from the training performed at the respective clinical site, to the central server. The central server may then average, or otherwise combine the updates in order update the central or "global" copy of the model.

Thus in this manner, the central server may select an optimally heterogeneous training dataset from available data across a plurality of clinical sites, with which to train a model, and train the model on the selected data in a distributed manner without the training data in the selected training dataset having to be transferred to the central server.

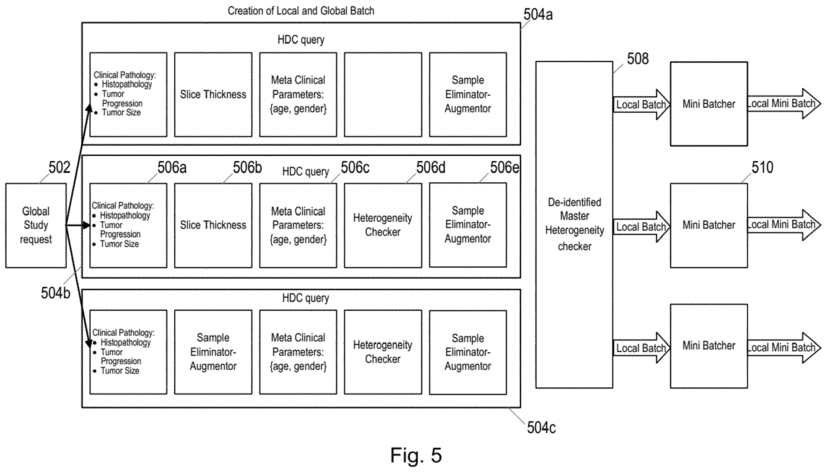

Fig. 5 illustrates a process according to an embodiment herein. In this embodiment, a global study request 502 is determined at a central sever (or "global node"). The global study request is sent to a plurality of clinical sites (or "local nodes" (HDC)) 504a, 504b, 504c. Although three clinical sites are shown in Fig. 5, it will be appreciated that this is an example only, and that the example may be generalized to any number of clinical sites. The global study request comprises a request that the clinical site sends metadata describing features of matching data at the respective clinical site that satisfies one or more clinical requirements 506a. The clinical requirements 506a comprise requirements for the data required for the model building, such as, for example:

1. Histopathology
2. Tumor Progression
3. Tumor Size, etc.

The clinical sites 504a, 504b, 504c enumerate the metadata satisfying the above requirements into a table 506c. Example metadata was given above and this example metadata applies equally to the embodiment of Fig. 5. Each clinical site 504a, 504b, 504c checks for the clinical requirements and also pulls out other features e.g. unspecified requirements 506b for distributed learning model building such as slice Thickness, age, gender etc. A second machine learning model 504d (e.g. heterogeneity checker) as described above with respect to Fig. 4, checks for local heterogeneity of the local batches of metadata (matching data at each clinical site). A sample eliminator-augmenter 506e removes unnecessary/irrelevant samples or adds additional data through augmentation techniques as per instruction of the heterogeneity checker 504d. Whether augmented data is needed or not may be determined from feedback on class imbalances from the heterogeneity checker and also from assessing the HDC ratios.

In this embodiment, a third machine learning model, or master heterogeneity checker 508 comprising the same type of machine learning model as the second machine learning model (e.g. the third machine learning model may be as described with respect to Fig. 4), shares all the metadata of the matching data (after proper de-identification) across all clinical sites and determines the heterogeneity of the training dataset as a whole. This helps in handling data imbalances. If a particular clinical site has fewer positive samples while another has more positive samples, this block allows us to carry forward with the training. If all clinical sites have less samples of a class (e.g. there is insufficient data in a class across all clinical sites), then augmentation may be performed (e.g. by block 506e) to supplement the training dataset.

The training data that is to be selected from each clinical site (e.g. the local batch at each clinical site) is finalized. Mini batches 510 may be formed (e.g. the local batches may be split up into mini-batches) depending on the computation capabilities of the local HDC. Preprocessing and training of the model then takes place on each clinical site and the results of the training are sent to the central server for combining (according to the distributed learning process).

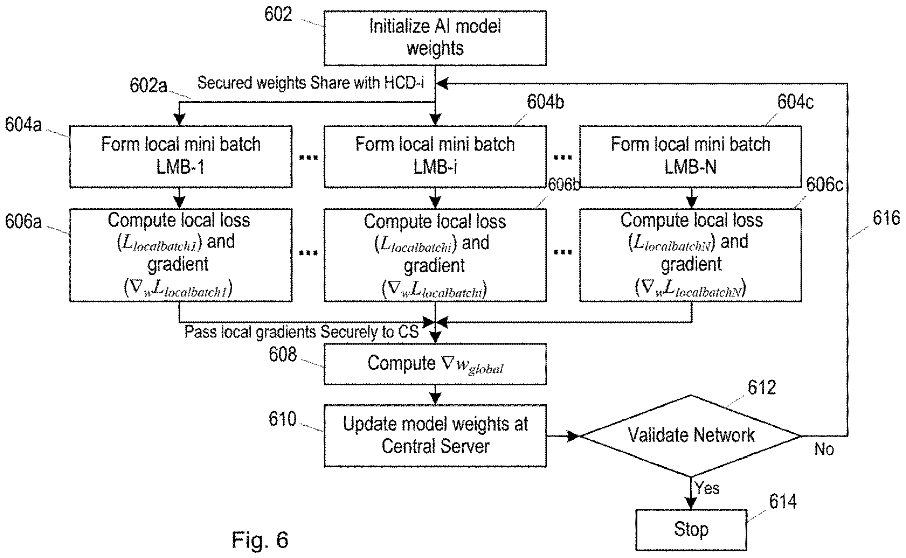

Fig. 6 illustrates a distributed learning process according to some embodiments herein. In this embodiment, a central server initializes model weights 602 and hyper-parameters and shares them 602a with a plurality of clinical sites. Three branches are illustrated in Fig. 6 corresponding to three clinical sites (the branches of steps performed by the three clinical sites are labelled a, b and c respectively), but it will be understood that this is just an example, and that the central server may send the initialized model weights to any number of clinical sites. Method 300 as described above is used to select 604a, 604b, 604c the training data that is to be used to train the model at each clinical site. In this embodiment, the selected data for each site is referred to as a "local batch". In combination, the local batches may be referred to as a "global batch".

Each clinical site trains 606a, 606b, 606c the model and computes the loss for its respective local batch and computes $\nabla_w L_{localbatch_i}$ using a gradient descent algorithm. The skilled person will be aware of methods for training a machine learning model to perform a task, using a local batch of training data. For example, the goal of a learning algorithm may be to minimize a loss function in systematic manner. For example, in the case of Bayesian networks weighted average of local boundaries may be computed; in the case of Support Vector Machines (SVM) weighted averages of global boundaries may be computed in proportion to the number of patients available at the hospital. In another example, in Deep Learning (including CNN, FCN, RNN), gradient descent based approaches may be used to minimize overall loss function to perform various tasks like classification, segmentation or regression. To perform gradient descent, concepts like SGD (stochastic gradient descent) may be used. A forward pass is performed using the selected local batch and a loss for the local batch is computed. In backward pass to minimize loss ($L_{localbatch}$), gradients are computed and weights are updated. Let, w be weights, to minimize total loss, we need to travel into the negative gradient ($\nabla_w L_{localbatch}$) direction with step size $\alpha$ shown in the equation below:

$$w : w \ - \ \alpha \nabla_w L_{localbatch}$$

To perform synchronous training using data from multiple sites, $\nabla_w L_{localbatch_i}$ are passed to the central server (CS). A global change in weight ($\nabla w_{global}$) is calculated 608, for example, using a weighted average with respect to the size of the local batch, e.g. according to an equation such as:

$$\nabla w_{global} = \sum_{i=1}^{n} \alpha_i * \nabla_w L_{local\ batch_i}$$

Weights may be updated, for example, according to:

$$w_{new} : w_{old} - \gamma * \nabla w_{global}$$

where $\gamma$ comprises a weighting applied to updates to the model.

At the end of each training epoch, the model performance may be validated 612 using validation data at the Central Server. If the validation is satisfied (e.g. the model meets a predetermined accuracy requirement), then the training ends. If the model does not satisfy the validation requirements, then the updated weights may be sent 616 to the clinical sites for further epoch(s) of training.

Thus there is described a method to train a model using a distributed learning process, by extracting relevant information from each data centres in the form of metadata. The disclosures herein can thus be used for medical imaging based diagnosis to train robust and generalized model with data available at multiple sites.

According to further embodiments, there is use of a model trained according to any of the methods described herein. For example, in some embodiments, there is use of a model trained according to the method 300 to classify or segment medical data such as medical images.

In another embodiment, there is provided a computer program product comprising a computer readable medium, the computer readable medium having computer readable code embodied therein, the computer readable code being configured such that, on execution by a suitable computer or processor, the computer or processor is caused to perform the method or methods described herein.

Thus, it will be appreciated that the disclosure also applies to computer programs, particularly computer programs on or in a carrier, adapted to put embodiments into practice. The program may be in the form of a source code, an object code, a code intermediate source and an object code such as in a partially compiled form, or in any other form suitable for use in the implementation of the method according to the embodiments described herein.

It will also be appreciated that such a program may have many different architectural designs. For example, a program code implementing the functionality of the method or system may be sub-divided into one or more sub-routines. Many different ways of distributing the functionality among these sub-routines will be apparent to the skilled person. The sub-routines may be stored together in one executable file to form a self-contained program. Such an executable file may comprise computer-executable instructions, for example, processor instructions and/or interpreter instructions (e.g. Java interpreter instructions). Alternatively, one or more or all of the sub-routines may be stored in at least one external library file and linked with a main program either statically or dynamically, e.g. at run-time. The main program contains at least one call to at least one of the sub-routines. The sub-routines may also comprise function calls to each other.

The carrier of a computer program may be any entity or device capable of carrying the program. For example, the carrier may include a data storage, such as a ROM, for example, a CD ROM or a semiconductor ROM, or a magnetic recording medium, for example, a hard disk. Furthermore, the carrier may be a transmissible carrier such as an electric or optical signal, which may be conveyed via electric or optical cable or by radio or other means. When the program is embodied in such a signal, the carrier may be constituted by such a cable or other device or means. Alternatively, the carrier may be an integrated circuit in which the program is embedded, the integrated circuit being adapted to perform, or used in the performance of, the relevant method.

Variations to the disclosed embodiments can be understood and effected by those skilled in the art in practicing the principles and techniques described herein, from a study of the drawings, the disclosure and the appended claims. In the claims, the word "comprising" does not exclude other elements or steps, and the indefinite article "a" or "an" does not exclude a plurality. A single processor or other unit may fulfil the functions of several items recited in the claims. The mere fact that certain measures are recited in mutually different dependent claims does not indicate that a combination of these measures cannot be used to advantage. A computer program may be stored or distributed on a suitable medium, such as an optical storage medium or a solid-state medium supplied together with or as part of other hardware, but may also be distributed in other forms, such as via the Internet or other wired or wireless telecommunication systems. Any reference signs in the claims should not be construed as limiting the scope.

**Claims**

1. A computer implemented method in a central server of selecting a training dataset with which to train a model using a distributed machine learning process, wherein the training dataset is to comprise medical data that satisfies one or more clinical requirements and wherein training data in the training dataset is located at a plurality of clinical sites, the method comprising:

   requesting (302) from each of the clinical sites, metadata describing features of matching data at the respective clinical site that satisfies the one or more clinical requirements;
   determining (304), from the metadata, a measure of variation of the features of the matching data; and
   based on the measure of variation, selecting (306) training data for the training dataset from the matching data using the metadata.

2. A method as in claim 1 wherein the step of selecting (306) training data for the training dataset from the matching data using the metadata comprises:
   selecting training data from the matching data at the plurality of clinical sites so as to increase the measure of variation of the features in the resulting training dataset.

3. A method as in claim 1 or 2 wherein the measure of variation of the features of the matching data is determined for matching data at each respective clinical site; and wherein the step of selecting training data for the training dataset comprises:
   selecting the training data from the matching data at the respective clinical site so as to increase the measure of variation of the training data selected from the respective clinical site.

4. A method as in claim 1, 2 or 3 wherein the measure of variation of the features of the matching data is determined for matching data across all of the plurality of clinical sites; and
   selecting the training data from the matching data across all the plurality of clinical sites so as to increase the measure of variation across the training dataset as a whole.

5. A method as in any one of the preceding claims further comprising:
   supplementing the training dataset with augmented training data so as to increase the variation of the selected training dataset.

6. A method as in any one of the preceding claims wherein the measure of variation comprises a measure of heterogeneity.

7. A method as in claim 6 wherein the measure of heterogeneity is determined using a second machine learning model that takes the features in the metadata as input and outputs the measure of heterogeneity.

8. A method as in claim 7 wherein the second machine learning model takes as input feature values in the metadata, $x_n$, and determines a combination of $x_n$ that optimally flattens a linear functi on $f(x)=x'\beta+b$, wherein $\beta$ comprises the gradient of the function and b comprises an offset.

9. A method as in claim 8 wherein the second machine learning model is configured to determine $f(x)$ with the minimal norm value ($\beta'\beta$) according to a convex optimization problem wherein the function:

$$J(\beta)=1/2(\beta'\beta);$$

is to be minimized.

10. A method as in claim 7, 8 or 9 wherein the second machine learning model comprises a support vector regression model.

11. A method as in any one of the preceding claims wherein the features in the metadata comprise different features to those specified in the clinical requirements.

12. A method as in any one of the preceding claims further comprising instructing each clinical site in the plurality of clinical sites to create a local copy of the model and train the local copy of the model using the training data in the training dataset selected from the respective clinical site; and
combining the results of the training according to the distributed learning process.

13. Use of a model trained according to the method of claim 12.

14. An apparatus for selecting a training dataset with which to train a model using a distributed machine learning process, wherein the training dataset is to comprise medical data that satisfies one or more clinical requirements and wherein training data in the training dataset is located at a plurality of clinical sites, the apparatus comprising:

a memory (104) comprising instruction data representing a set of instructions; and
a processor (102) configured to communicate with the memory and to execute the set of instructions, wherein the set of instructions, when executed by the processor, cause the processor to:

request from each of the clinical sites, metadata describing features of matching data at the respective clinical site that satisfies the one or more clinical requirements;
determine, from the metadata, a measure of variation of the features of the matching data; and
based on the measure of variation, select training data for the training dataset from the matching data using the metadata.

15. A computer program product comprising a computer readable medium, the computer readable medium having computer readable code embodied therein, the computer readable code being configured such that, on execution by a suitable computer or processor, the computer or processor is caused to perform the method as claimed in any one of claims 1 to 12.

Fig. 1

200

202 — Processor

204 — Memory

Fig. 2

300

Request from each of the clinical sites, metadata describing features of matching data at the respective clinical site that satisfies the one or more clinical requirements

302

Determine, from the metadata, a measure of variation of the features of the matching data

304

Based on the measure of variation, select training data for the training dataset from the matching data using the metadata

306

Fig. 3

404

Other
HDC
balance
ratios

406

402

Queried Samples

Heterogeneity
Checker

408

H-Score

Fig. 4

Fig. 5

602 — Initialize AI model weights

602a — Secured weights Share with HCD-i

604a — Form local mini batch LMB-1  ...  604b — Form local mini batch LMB-i  ...  604c — Form local mini batch LMB-N

606a — Compute local loss $(L_{localbatch1})$ and gradient $(\nabla_w L_{localbatch1})$  ...  606b — Compute local loss $(L_{localbatchi})$ and gradient $(\nabla_w L_{localbatchi})$  ...  606c — Compute local loss $(L_{localbatchN})$ and gradient $(\nabla_w L_{localbatchN})$

616

Pass local gradients Securely to CS

608 — Compute $\nabla w_{global}$

610 — Update model weights at Central Server

612 — Validate Network

No

Yes

614 — Stop

Fig. 6

17

Europäisches
Patentamt

European
Patent Office

Office européen
des brevets

## EUROPEAN SEARCH REPORT

Application Number

EP 20 18 6331

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2002/169652 A1 (BUSCHE FREDERICK D [US]) 14 November 2002 (2002-11-14) * paragraph [0033] - paragraph [0103] * * figure 6 * ----- | 1-15 | INV. G06N3/00 |
| A | US 2016/132787 A1 (DREVO WILL D [US] ET AL) 12 May 2016 (2016-05-12) * paragraph [0006] - paragraph [0020] * ----- | 1,13-15 | |
| A | V. R. ELGIN CHRISTO ET AL: "Feature Selection and Instance Selection from Clinical Datasets Using Co-operative Co-evolution and Classification Using Random Forest", JOURNAL OF THE INSTITUTION OF ELECTRONICS ANDTELECOMMUNICATION ENGINEERS., 28 January 2020 (2020-01-28), pages 1-14, XP055756171, ISSN: 0377-2063, DOI: 10.1080/03772063.2020.1713917 * the whole document * ----- | 1,13-15 | |

TECHNICAL FIELDS
SEARCHED      (IPC)

G06N
G16H

The present search report has been drawn up for all claims

| Place of search | Date of completion of the search | Examiner |
|---|---|---|
| Munich | 3 December 2020 | Rinelli, Pietro |

EPO FORM 1503 03.82 (P04C01)

## ANNEX TO THE EUROPEAN SEARCH REPORT
## ON EUROPEAN PATENT APPLICATION NO.

EP 20 18 6331

This annex lists the patent family members relating to the patent documents cited in the above-mentioned European search report.
The members are as contained in the European Patent Office EDP file on
The European Patent Office is in no way liable for these particulars which are merely given for the purpose of information.

03-12-2020

| Patent document cited in search report | Publication date | Patent family member(s) | Publication date |
|---|---|---|---|
| US 2002169652 A1 | 14-11-2002 | NONE | |
| US 2016132787 A1 | 12-05-2016 | US 2016132787 A1 | 12-05-2016 |
| | | WO 2016077127 A1 | 19-05-2016 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

• **BONAWITZ et al.** *Towards Federated Learning at Scale: System Design,* 2019